# EUROPEAN PATENT SPECIFICATION

(11) **EP 1 101 494 B1**
(45) Date of publication and mention of the grant of the patent: **15.12.2004**
(21) Application number: 99934451.8
(22) Date of filing: 28.07.1999
(51) Int. Cl.: A61K 35/50, A61K 7/48, A61P 17/00

(54) **Composition for stimulating the synthesis of the melanic pigment and procedure to obtain it**
ZUSAMMENSETZUNG ZUR STIMULIERUNG DER MELANINPIGMENT-SYNTHESE UND VERFAHREN ZU DEREN HERSTELLUNG
COMPOSITION POUR LA STIMULATION DE LA SYNTHESE DU PIGMENT MELANIQUE ET SON PROCEDE D'OBTENTION

(30) Priority: 28.07.1998 CU 11098
(43) Date of publication of application: 23.05.2001
(73) Proprietor: Centro de Histoterapia Placentaria, La Lisa, Ciudad de la Habana 17100 (CA)
(72) Inventor: MIYARES CAO, Carlos Manuel, Playa, Ciudad de la Habana 11300 (CU)
(74) Representative: Prins, Adrianus Willem, Mr. Ir.
(86) International application number: PCT/CU1999/000003
(87) International publication number: WO 2000/006180

(56) References cited:
- EP-A- 0 839 535
- FR-A- 2 537 585
- US-A- 4 507 277
- JIMBOW K.: "Vitiligo. Therapeutic advances." DERMATOLOGIC CLINICS, vol. 16, no. 2, April 1998 (1998-04), pages 399-407, XP002122297
- K. U. SCHALLREUTER ET AL.: "Treatment of vitiligo with a topical application of..." DERMATOLOGY, vol. 190, no. 3, 1995, pages 223-229, XP002122298
- C. J. CARSBERG ET AL.: "Intracellular calcium modulates the responses..." JOURNAL OF DERMATOLOGICAL SCIENCE, vol. 9, no. 3, May 1995 (1995-05), pages 157-164, XP002122299

## Description

### Technical sector

The present invention is related to the branch of the Human Medicine, and with the Dermatology, and in particular to a composition developed for stimulating the synthesis of the melanic pigment of the skin, and therefore useful in the treatment of the vitiligo, as well as to the procedure for obtaining this composition.

### Prior Art

The vitiligo or leucoderm is an illness of the oldest known by the humanity, that is characterized by the loss of the cells that produce of the melanic pigment of the skin. This illness affects approximately 1% of the world's population, without distinguishing age, sex or race. It is of ethiology unknown and without a defined therapy until the moment, and it is characterized by a gradual depigmentation of the skin of patients subjected to situations of extreme nervous tension, which affects their psyche and social behavior unfavorably once their external symptoms begin, under the aspect of areas of white skin surrounded by a hyperpigmentated halo, showing fundamentally in the face, trunk or articular regions.

It is known from the state of the art that certain chemical substances of vegetable origin or semisynthetically produced, denominated psoralens, can be used for the treatment of the vitiligo. These substances, when being administered by oral or topical route, concentrate on the melanocytes or dermal pigment-producing cells, absorbing the energy coming from the ultraviolet radiation and promoting the production of this pigment, the melanin (Arnold, M. J. Jr. "Psoralens and Suntan Hawaii Med. J. 1957-16391); (Becker, S.W. Jr. "Methods of increasing skin pigmentation J. Sec. Cosmetic Ehem. 1958-9-80); (Fitzpatrick, T.B. Pigmentary Diseases Current Therapy 1958-314); (Becker, S.W. Jr. "Effects of 8-Methoxy Psoralen and ultraviolet light in human skin". Science 1958-127-878); (Sidi, E. Planat, P. Practical considerations on current treatment of vitiligo Revue of Medicins, 1968-23 Dec).

However, the psoralens are not exempt of secondary toxic effects, producing dermatitis and necrosis fo the skin when being applied by topical route. Also, their effect is slow and reversible in most of the cases when discontinuing the medication.

On the other hand, these substances present difficulties for their application, which requires the gradual increment of the daily time of exposition of the patient to the ultraviolet radiation, causing in occasions severe toxic reactions such as burns due to their topical administration or hepatic failure, digestive, renal or nervous disorders with their administration by oral. route.

The known nearer similar technical solution to the present invention is the Melagenin Lotion®, Patent. FR 8220746.

The Melagenin Lotion® is an alcoholic extract to 50% of human placenta, which is obtained from healthy pregnat women under aseptic conditions after normal childbirths. This extract contains a lipoprotein of molecular weight among 1500 - 4000 Daltons, which constitutes its active principle.

This substance stimulates the reproduction of the melanocytes and the synthesis of the melanic pigment, also accelerating the oxidation of the amino acid L-dopa in presence of solar light, which favors its transformation in melanin after internal chemical processes.

The Melagenin Lotion® has been used successfully until the moment for the treatment of vitiligo. According to the clinical trials carried out, their use has reached a remarkable effectiveness in all the patients where it has been applied (Miyares Cao C., Taboas M. y López H. "Informe preliminar sobre el empleo de extracto placentario humano en la terapéutica del Vitiligo". Revista Cubana de Farmacia 10 (1). 1976; Miyares Cao C. y colaboradores. Estudio experimental y clínico del efecto pigmentante epidérmico del extracto placentario humano". Revista Cubana de Farmacia. Volumen 20, No. 6, Noviembre-Diciembre 1981; Sharma S. K.,. Jain R. K and Sharma A. K. Topical human placental extract for the treatment of Vitiligo, A preliminary study; Miyares C. Y colaboradores: Estudio experimental y clínico del efecto pigmentante epidérmico del extracto placentario humano. Annais Brasileiros de Dermatología. 1986: 61: 3 (suplemento); Miyares Cao C. Melagenina Producto Cubano. Nuevo y Eficaz medicamento para el tratamiento del Vitiligo. Serie de Reseñas Nacionales. Ed. Palacio de las Convenciones. La Habana, Pag. 15. 1986).

The Melagenin Lotion@, although it lacks collateral noxious effects, is a product that has to be used in three daily applications with interval among them of 8 hours, and accompanied by solar or infrared exposition in one of the applications. All this hinders the treatment compliance in most patients.

Similarly it is known from the state of the art that calcium has an active participation in the process of pigmentation of the skin, by means of the stimulation of the secretory activity of the melanocytes, in connection with the concentration of this ion (Meyer, A. 1986. Influence of calcium on the melanocytes in the inner ear. Abst; 13th International Pigment Cell Conference Oct. 5-6, Tucson, Arizona, USES; and Negishi, S. 1986. The role of calcium in light response of onyzias melonophores. Abst; 13th International Pigment Cell Conference Oct. 5-6, Tucson, Arizona, USES).

### Disclosure of the invention

The novelty of the present invention consists on a new composition containing the same active principle of the Melagenin Lotion®, in this case an alphalipoprotein obtained from the placental human cotyledons, by means of the treatment of these with organic solvents, and their later redisolution in a solution of ethanol with calcium chloride, which allows that a product is obtained that shows a synergic effect between both components on its pigmenting effect, decreasing the number of applications of this product from 1 every 8 hours for patient, to 1 every 24 hours, being also eliminated the necessity to expose the patient to solar or infrared radiations after any of the applications.

The new product obtained, denominated from this point Melagenin Plus can be used in a single daily application, which constitutes the current practice in the world therapy.

This product stimulates the synthesis of the melanic pigment of the skin and the reproduction of the melanocytes as it is demonstrated by the pharmacological trials executed, lacking any severe secondary reactions as shown in the toxicological, teratological and clinical trials carried out using it.

This product is of easy obtaining and application, and the re-pigmenting begins quickly (15 to 20 days) after beginning the treatment, being irreversible after discontinuing the application of the product. The color acquired by the skin where it is applied is identical to that of the areas of the patient's normal skin, which in turn don't increase the intensity of their coloration later on.

As the matter from where the active principle of the product is extracted is of human origin, there are no immunologic reactions to fear, with no contraindications related to age, sex or race for its use.

### Obtaining of the active principle of the composition Melagenin Plus.

For the obtaining of the active principle of the present invention it was carried out the following procedure:

A kilogram of placental cotyledons was used after they were let to freeze during 7 to 10 days, then they were crushed and macerated with ethanol from 90 to 96° to equal parts weight/volume. Then, this macerated is filtered through dense gauze, leaving it to rest during 24 hours.

It was determined the pigmenting activity to the supernatant which was positive after 20 to 50 days of being used in topical form on ears of mouse Balb/C 57 BL 6.

To 200 ml of this extract with the pigmenting factor are added by means of a hypodermic needle 10 ml of a saturated solution of benzoic acid in ethanol from 90 to 96°, made dissolving from 2 to 8 g of benzoic acid in 5 to 12 ml of ethanol of 90 to 96°.

The mixture was agitated from 10 to 50 minutes and it was filtered with Whatman filter paper No. 2 to 5. Then it was washed the precipitate obtained with 200 to 500 ml of a saturated solution of benzoic acid in distilled water, by dissolving from 2 to 10 g of benzoic acid in 300 to 1000 ml of distilled water.

Afterwards the precipitate was removed from the filter paper and they were added from 100 to 500 ml of 30% acetone. Then it was centrifuged to 1500 rpm during 10 to 30 minutes, being discarded the supernatant.

The precipitate was again washed for 2 to 10 times more with 100 at 500 ml of acetone, drying off to the vacuum and to room temperature. The product was re-dissolved in alcohol from 90 to 96° containing 0.2 to 4.0 mg/ml of calcium chloride.

Five ml of the above product with biological activity was filtered through Sephadex G-30 or 100, using buffer sodium phosphate 5 x 10⁻¹ to 5 x 10⁻⁶ moles, pH = 4 at 8, which was packed in a column from 1 to 3 cm width and from 18 to 40 cm of height. The flow was from 10 to 30 drops for minute and the collected fractions from 5 to 10 ml each one.

Two proteic peacks were obtained, the first one of 100 to 300 µg/ml and the second one of 20 to 150 µg/ml. The pigmenting activity was positive for the second peack, appearing within the 10 to 20 days after being used topically the lyophilized mixture in the guinea pig.

The lyophilized mixtures of both proteic peacks were run in electrophoresis with polyacrilamide gel and in agarose, staining the first one with a coloring for proteins (amido black) and the second to detect lipids (sudan). In the electrophoresis of proteins it was observed a colored small band followed by another without coloring, both of little migration; a very visible band was also noticed in the region of albumin. In the electrophoresis of lipids, a band was observed in the region wherein the alphalipoproteins migrate.

### Synergic pigmenting effect of the Melagenin and Calcium.

The melanocitopoyetic activity of the resulting product was evaluated by topical application of the Melagenin Plus during a period of time of 5 serial days in a daily occasion on the epidermis of the ears of black mice of the strain B6 D2. For the trial 60 black mice separated in 4 groups denominated A, B, C and D were used and integrated by 5 mice each one, to which were applied the following treatments.

| Group | Daily topical treatment |
|---|---|
| A | 70% Alcohol, as vehicle of the Melagenin® (Control) |
| B | 70% Alcoholic solution with addition of calcium chloride, concentration 1 mg/ml. |
| C | Melagenin Lotion® |
| D | Melagenin Lotion® with addition of calcium chloride at a concentration of 1 mg/ml (Melagenin Plus) |

The alcohol at 70%, the alcoholic solution at 70% with calcium chloride 1 mg/ml, as well as the Melagenin Lotion® and the Melagenin Plus were applied by rubbing the product with the fingers of the operator on the ears of the animals.

Concluded the time of treatment is allowed to lapse 48 hours and the animals are sacrificed in order to extract them the epidermis of the ears and to subject them to the histochemical technique of the L-dopa oxidase that allows, by means of the microscopic observation, the melanocyte count for mm² in this histologic structure.

To determines the number of melanocytes for mm², 5 different areas were selected from the densely populated zone of each ear, and using a particular program elaborated for this end (but in this case the specific section for count of cells) the number of cells was determined by mm² in each one of the 5 areas so that 150 melanocytes values are obtained by mm² per each group (15 animals per group and 10 counts per animal each).

The values obtained in the melanocyte count by mm² were subjected to analysis of variance of simple classification and with posteriority to a test of Kruskal-Wallis and test Student Newman-Keuls (SNK), in order to determine significant differences existing among these groups.

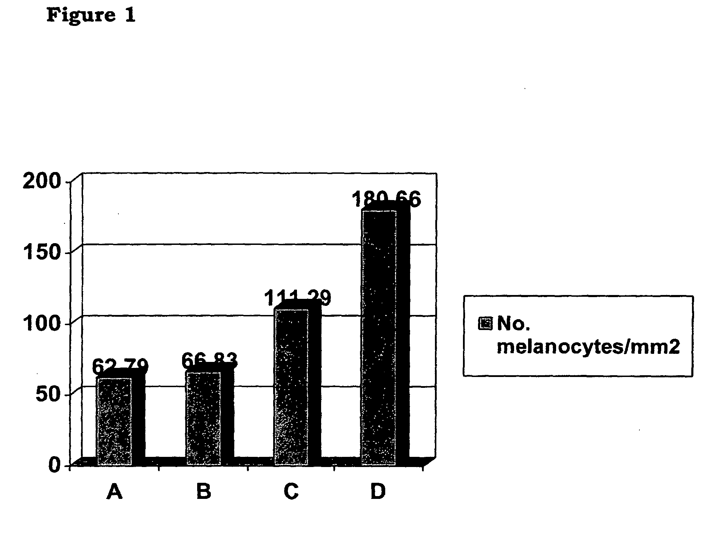

The results obtained by means of this experience can be observed in the Table I and being graphically shown in the Figure 1.

**Table I**

| Test of Kruskal-Wallis | |
|---|---|
| Quantity of melanocytes / mm² | |
| **X** | **N** |
| 180.66 a | 150 |
| 111.29 b | 150 |
| 66.83 c | 150 |
| 62.79 d | 150 for p< 0.001 |

| | |
|---|---|
| a= Melagenin Plus | |
| b= Melagenin Lotion® | |
| c= Alcohol 70 % + Calcium 1 mg/ml | |
| d= Alcohol 70 % | |

It was observed that for p <0.001, a significant increment of the number of melanocytes/mm² existed in the epidermis of the ears of the black mice treated with Melagenin Lotion® and Melagenin Plus, in respect with the groups treated with alcohol and without treatment.

The increment of the melanocyte number was bigger in the animals treated with Melagenin Plus.

Other differences found were:
- There is a significant difference among the results obtained using Melagenin Plus and using Melagenin Lotion® only.
- There is a significant differences among the results obtained using Melagenin Plus and using alcohol plus calcium only.
- There is a significant difference among the results obtained using Melagenin Plus and using alcohol only.
- There is a significant difference among the results obtained using Melagenin Lotion® and using alcohol plus calcium.
- There is a significant difference among the results obtained using Melagenin Lotion® and the Control group treated with alcohol only.
- It doesn't exist significant difference between the Control group and the group treated with alcohol plus calcium.

Additionally it was proven that when the Melagenin Lotion® and the Melagenin Plus are applied indistinctly in biological trials, it was observed that in the case of the Melagenin Plus the pigmenting effect in nipples of male guinea pigs is only visible in 13 days, while with the Melagenin Lotion® this happens at day 18. Similarly, in the tale of the black mice, a bigger melanin concentration it was observed using Melagenin Plus when the cuts are treated by the histochemical method, where it was observed to a bigger increment of the melanocytes in the basal region of the epidermis.

### Irritation studies in the skin of animals.

The product Melagenin Plus was applied to a group of animals in an area of 42 cm² with a dose of 20 mg/cm² and in that same animal the solvent was applied.

During the two weeks of the experiment it was not observed any type of irritation or affectation of the skin. The histopathologic studies do not reflect damage.

In conclusion it can be pointed out that the product Melagenin Plus doesn't produce irritation in the skin.

### Clinical trials in humans.

To carry out these trials, 30 vitiligo patients were selected randomly.

The patients were separated alternatively in two groups denominated I and II, and integrated by 15 patients each one.

Those belonging to the group I were indicated to use Melagenin Lotion® (Alcoholic Extract of Human Placenta at 50%) applying it topically by means of finger rubbing on the areas of skin depigmentated by the illness, in three daily occasions with intervals of 8 hours (6.00 am, 2.00 pm and 10.00 pm).

In one of these occasions the treated areas should be exposed to lamps of infrared light of 250 watts of intensity, placed at a distance of 40 cm, during 15 minutes. The application of the medication should be repeated every 5 minutes in that interval of time.

In turn the members of the group II would use Melagenin Plus (Alcoholic extract of human placenta at 50% containing calcium chloride) applying it in a similar way but in a single daily occasion, without the exposure to the infrared radiation and with intervals of 24 hours.

The participants in the trial should remain before its beginning without any specific treatment.

The patients object of the experience were evaluated monthly during a period of time of 6 months, fixed to compare in both groups the repigmentation degree obtained with each product, as well as the development of secondary reactions.

They were practiced biopsies of areas of skin depigmented to the beginning and final of the experience in 5 patients of each group selected randomly.

Histological technique of L-dopa Oxidase (Negishi, S. 1986. The role calcium in light response of onyzias melanophores. Abst; 13th International Pigment cell Conference oct 5-6 Tucson, Arizona, USES) was used to identify the melanocytes in the same ones.

Jointly the differences were determined in the consumption of flasks of the medication in each group.

For the procedure of the statistical analysis of the percentage of reduction of the depigmented corporal surface in each group, as well as for the determination of the same for patient, a program was used elaborated with such objective (Coyula, R. Automatizacion de la Creación de Historia Clínica de los enfermos de Vitiligo. 1ra Conferencia Latinoamericana de Aplicaciones de la Matemática y la Computación a la Biologia. CENIC, 31 Oct. - 30 Nov. 1991, La Habana, Cuba).

The results obtained by means of this clinical trial are summarized in Tables II, III, IV and V, in which they have been considered as the patients' data the number of their clinical history for their identification, the age, the sex, the race and the years that the have suffered of the illness.

Among the parameters that have been evaluated they are the % of depigmentation at the beginning and at the end of the treatment, the % of pigmentation reached and the flasks of the medication consumed during this treatment concluding. Likewise, the analyzed areas corresponding to each one of this patients treated (face, neck, trunk, extremities or genitals) are shown in the table pertaining to each treatment type.

**TABLE II:**

| Results reached using the Melagenin Lotion® | | | | | | | | |
|---|---|---|---|---|---|---|---|---|
| PATIENT CH No. | AGE (years) | SEX | RACE | YEARS OF ILL | % DEP. | | % PIGM. | FLASKS |
| | | | | | BEGIN | END | | |
| 255 | 13 | M | N | 12 | 26 | 16 | 10 | 18 |
| 268 | 14 | F | B | 3 | 8 | 3 | 5 | 6 |
| 277 | 12 | F | B | 2 | 5 | 2 | 3 | 6 |
| 286 | 5 | F | B | 2.6 | 6 | 0 | 6 | 6 |
| 292 | 10 | F | B | 3 | 24 | 21 | 3 | 18 |
| 293 | 56 | F | B | 12 | 11 | 9 | 2 | 12 |
| 381 | 34 | F | B | 10 | 23 | 16 | 7 | 18 |
| 591 | 21 | F | M | 11 | 59 | 39 | 20 | 98 |
| 674 | 5 | M | M | 3 | 58 | 30 | 28 | 48 |
| 771 | 4 | M | B | 0.2 | 8 | 5 | 3 | 6 |
| 849 | 44 | F | N | 10 | 13 | 8 | 7 | 12 |
| 838 | 5 | F | B | 0.6 | 8 | 2 | 6 | 6 |
| 852 | 5 | M | N | 3.5 | 43 | 32 | 11 | 48 |
| 1021 | 12 | F | M | 3 | 8 | 4 | 4 | 6 |
| 1044 | 27 | M | B | 5 | 9 | 3 | 6 | 6 |

**TABLE III:**

| Areas analyzed in patients treated with Melagenin Lotion® | | | | | | | |
|---|---|---|---|---|---|---|---|
| AREAS AFFECTED BY VITILIGO | | | | | | | |
| PATIENT CH No. | FACE | NECK | TRUNK | | EXTREMITIES | | GENITALS |
| | | | FRONT | BACK | SUP. | INF. | |
| 255 | X | | X | | X | X | X |
| 268 | X | | | | | X | |
| 277 | X | | X | | X | | |
| 286 | | | X | X | | | |
| 292 | X | | | | X | X | |
| 293 | X | | | | X | X | |
| 381 | X | | X | | X | X | X |
| 591 | | X | X | | X | X | |
| 674 | | | | X | X | X | |
| 771 | | | X | X | | X | |
| 849 | | | | | | X | |
| 838 | X | | X | X | | X | |
| 852 | X | | X | | X | X | |
| 1021 | X | | | | | X | |
| 1044 | | | | | X | X | |

**TABLE IV:**

| Results reached using the Melagenin Plus. | | | | | | | | |
|---|---|---|---|---|---|---|---|---|
| PATIENT CH No. | AGE (years) | SEX | RACE | YEARS OF ILL | % DEP. | | % PIGM. | FLASKS |
| | | | | | BEGIN | END | | |
| 2 | 20 | M | M | 1 | 2 | 0.5 | 1.5 | 1 |
| 31 | 1 | F | B | 5 | 6 | 2 | 4 | 2 |
| 126 | 5 | F | N | 0.2 | 1 | 0.5 | 0.5 | 1 |
| 154 | 9 | F | B | .5 | 39 | 7 | 22 | 8 |
| 219 | 3 | F | B | 8 | 40 | 7 | 13 | 8 |
| 231 | 8 | F | B | 9 | 11 | 3 | 8 | 4 |
| 341 | 3 | M | B | 1 | 4 | 2 | 2 | 2 |
| 486 | 5 | F | N | 2 | 4 | 1 | 3 | 2 |
| 491 | 5 | F | M | 4 | 24 | 1 | 3 | 6 |
| 733 | 5 | F | B | 3 | 13 | 0 | 3 | 4 |
| 779 | 3 | F | N | 0.6 | 4 | 2 | 2 | 2 |
| 781 | 3 | M | B | 23 | 6 | 3 | 3 | 2 |
| 888 | 7 | M | B | 0.2 | 12 | 6 | 6 | 4 |
| 1096 | 6 | F | B | 4 | 14 | 0 | 4 | 4 |
| 1116 | 4 | M | B | 1 | 22 | 2 | 10 | 6 |

**TABLE V:**

| Areas analyzed in patients treated with Melagenin Plus | | | | | | | |
|---|---|---|---|---|---|---|---|
| AREAS AFFECTED BY VITILIGO | | | | | | | |
| PATIENT CH No. | FACE | NECK | TRUNK | | EXTREMITIES | | GENITALS |
| | | | BEGIN | END | SUP. | INF. | |
| 2 | X | | | | | | |
| 31 | | | | | X | X | |
| 126 | | | | | | | X |
| 154 | X | | X | | X | X | X |
| 219 | X | | X | X | X | X | |
| 231 | X | | | | X | X | X |
| 341 | | | | | | X | |
| 486 | | | | | X | | |
| 491 | X | | X | | X | X | |
| 733 | | | X | | | X | |
| 779 | | | | X | X | | |
| 781 | | | | | | X | X |
| 888 | X | | | X | | | |
| 1096 | X | X | | | X | | X |
| 1116 | | | | | X | | X |

Analyzing the previous tables it can be observed that after having concluded the 6 months of treatment, an average value of 59.11% of decrease of the body surface depigmented by the illness is appreciated in the group treated with Melagenin Lotion® and of 50.79% in that treated with Melagenin Plus, without being appreciated any significant difference between both results, for a value p <0.05.

They didn't observed local or systemic secondary reactions in the patients from the groups subjected to the trial.

The biopsies practiced in the 5 patients of each group showed the reappearance of the melanocytes in the vitiligo areas selected for the histologic study pre and post treatment.

Finally, a bigger consumption of medication flasks was observed in the group treated with Melagenin Lotion® with a total of 264 and an average of 17,6 flasks for patient in the 6 months that the experience lasted, regarding the group treated with Melagenin Plus, in the one which alone 56 flasks were used, with an average of 3.7 flasks for patient in same time.

The histological study carried out demonstrated that the re-pigmenting effect of both products on the areas affected by vitiligo is due to that they induce again the reproduction of the melanocytes in these places.

The absence of reactions systematic local secondary reaffirms the innocuoity of both products.

The synergic action of the Calcium and the Melagenin facilitates the execution of the treatment on the part of the vitiligo patients, decreasing the number of daily applications of the product and consequently the consumption of flasks in each treatment. Also the new formulation allows that the necessity of exposing the patient to solar or infrared radiations after the application is eliminated.

### Brief description of the drawings.

Figure 1 shows the melanocytopoyetic action obtained through an assay of ear of black mice in 4 groups denominated A, B, C and D, to which were applied the following treatments:

| Group | Daily topical treatment |
|---|---|
| A | 70% Alcohol, as vehicle of the Melagenin® (Control) |
| B | 70% Alcoholic solution with addition of calcium chloride, concentration 1 mg/ml. |
| C | Melagenin Lotion® |
| D | Melagenin Lotion® with addition of calcium chloride at a concentration of 1 mg/ml (Melagenin Plus) |

## Claims

1. Process for obtaining a composition for stimulating synthesis of melanic pigment comprising the steps of:
- crushing a kilogram of frozen placental cotyledons and macerating it with ethanol from 90 to 96° to equal parts weight/volume,
- filtering the macerated leaving it to rest during 24 hours,
- adding to 200 ml of the extract containing the pigmenting factor a saturated solution of benzoic acid in ethanol from 90 to 96°,
- agitating the mixture from 10 to 50 minutes and filtering it,
- washing the precipitate obtained with a saturated solution of benzoic acid in distilled water,
- removing the precipitate and adding acetone 30% to it,
- centrifuging the extract to 1500 rpm during 10 to 30 minutes and discarding the supernatant,
- washing the precipitate again with acetone and drying off to vacuum at room temperature,
- redissolving the product in alcohol at from 90 to 96° containing calcium chloride in a concentration from 0.2 to 4.0 mg/ml,
- filtering the product obtained through a column of Sephadex using buffer phosphate of sodium pH= 4 to 8
- collecting the fractions corresponding to the second protein peak of 20 to 150 µg/ml,
- lyophilising, and
- redissolving in an alcoholic solution at 50% containing CaCl₂ at a concentration of 0.2 to 4.0 mg/ml.

2. Composition for stimulating synthesis of melanic pigment comprising a mixture of alcoholic extract of human placenta processed with ethanol in water at 90-96%, which mixture contains an alpha-lipoprotein of molecular weight between 1500 and 4000 daltons and calcium chloride in a concentration between 0.2 and 4.0 mg/ml.

3. Use of a composition according to claim 2 for the manufacture of a medicament useful in the treatment of vitiligo wherein this mixture is adapted to be applied topically to a patient by means of rub with the fingers in the affected areas as an only daily application, without the necessity of exposition thereof to infrared radiation and during a period of treatment between 6 months and one year, depending on the affectation of the illness.

## Patentansprüche

1. Verfahren zur Herstellung einer Zusammensetzung zur Stimulierung der Synthese des Melaminpigmentes, umfassend die Schritte:
- Zerkleinern von 1 kg gefrorenen Kotyledonen von der Plazenta und deren Zersetzen mit Ethanol mit 90 bis 96° zu gleichen Teilen (Gewicht/Volumen),
- Filtern des Zersetzungsproduktes, wobei es für 24 Stunden ruhen kann,
- Zugeben einer gesättigten Lösung von Benzoesäure in Ethanol mit 90 bis 96° zu 200 ml des den Pigmentierungsfaktor enthaltenden Extraktes,
- Rühren des Gemischs für 10 bis 50 Minuten und Filtern des Gemischs,
- Waschen des erhaltenen Niederschlags mit einer gesättigten Lösung von Benzoesäure in destilliertem Wasser,
- Entfernen des Niederschlags und Zugeben von 30%igem Aceton,
- Zentrifugieren des Extraktes für 10 bis 30 Minuten mit bis zu 1500 U/min und Weggießen des Überstandes,
- erneutes Waschen des Niederschlags und Vakuumtrocknen bei Raumtemperatur,
- erneutes Auflösen des Produktes in Alkohol bei 90 bis 96°, der Calciumchlorid in einer Konzentration von 0,2 bis 4,0 mg/ml enthält,
- Filtern des erhaltenen Produktes durch eine Sephadex-Säule, wobei Natriumphosphat-Puffer mit pH = 4 bis 8 verwendet wird,
- Auffangen der Fraktionen, die dem zweiten Protein-Peak bei 20 bis 150 µg/ml entsprechen,
- Gefriertrocknen und
- erneutes Auflösen in einer Alkohollösung mit 50 %, die CaCl₂ in einer Konzentration von 0,2 bis 4,0 mg/ml enthält.

2. Zusammensetzung zur Stimulierung der Synthese des Melaminpigmentes, umfassend ein Gemisch eines Alkoholextraktes der Human-Plazenta, der mit Ethanol in Wasser mit 90 bis 96 % behandelt worden ist, wobei das Gemisch ein α-Lipoprotein mit einem Molekulargewicht von 1500 bis 4000 Dafton und Calciumchlorid in einer Konzentration von 0,2 bis 4,0 mg/ml enthält.

3. Verwendung einer Zusammensetzung nach Anspruch 2 für die Herstellung eines Medikamentes, das bei der Behandlung von Vitiligo vorteilhaft ist, wobei das Gemisch für die topische Anwendung bei einem Patienten durch Einreiben der betroffenen Bereiche mit den Fingern als einzige tägliche Anwendung, ohne daß die Bereiche IR-Strahlen ausgesetzt werden müssen, und in Abhängigkeit vom Zustand der Erkrankung für einen Behandlungszeitraum von 6 Monaten bis zu einem Jahr geeignet ist.

## Revendications

1. Procédé d'obtention d'une composition permettant de stimuler la synthèse de pigment mélanique, comprenant les étapes consistant à :
- broyer un kilogramme de cotylédons placentaires congelés et le faire macérer avec de l'éthanol entre 90 et 96° en parties en poids/volume égales,
- filtrer la masse macérée en la laissant reposer pendant 24 heures,
- ajouter à 200 ml de l'extrait contenant le facteur de pigmentation une solution saturée d'acide benzoïque dans de l'éthanol entre 90 et 96°,
- agiter le mélange pendant 10 à 50 minutes et le filtrer,
- laver le précipité obtenu avec une solution saturée d'acide benzoïque dans de l'eau distillée,
- retirer le précipité et lui ajouter de l'acétone 30 %,
- centrifuger l'extrait à 1 500 tpm pendant 10 à 30 minutes et éliminer le surnageant,
- laver à nouveau le précipité avec de l'acétone et sécher sous vide à la température ambiante,
- re-dissoudre le produit dans un alcool entre 90 et 96° contenant du chlorure de calcium dans une concentration comprise entre 0,2 et 4,0 mg/ml,
- filtrer le produit obtenu par une colonne de Sephadex en utilisant un tampon phosphate de sodium, pH 4 à 8,
- recueillir les fractions correspondant au second pic de protéines de 20 à 150 µg/ml,
- lyophiliser, et
- re-dissoudre dans une solution alcoolique à 50 % contenant CaCl₂ selon une concentration de 0,2 à 4,0 mg/ml.

2. Composition pour stimuler la synthèse de pigment mélanique comprenant un mélange d'extrait alcoolique de placenta humain traité avec de l'éthanol dans de l'eau à 90-96 %, lequel mélange contient une alpha-lipoprotéine de masse moléculaire comprise entre 1 500 et 4 000 daltons et du chlorure de calcium selon une concentration comprise entre 0,2 et 4,0 mg/ml.

3. Utilisation d'une composition selon la revendication 2 pour fabriquer un médicament utile dans le traitement du vitiligo, dans lequel ce mélange est adapté pour être appliqué topiquement à un patient en frottant avec les doigts les zones affectées sous la forme d'une application quotidienne, sans qu'il soit nécessaire de les exposer à un rayonnement infrarouge et pendant une période de traitement comprise entre 6 mois et un an, en fonction du degré d'affection de la maladie.
